# EUROPÄISCHE PATENTANMELDUNG

(11) **EP 4 796 075 A1**
(43) Veröffentlichungstag der Anmeldung: **26.08.2026**
(21) Anmeldenummer: 26151471.5
(22) Anmeldetag: 13.01.2026
(51) Int. Cl.: A61C 19/00, A61B 90/70, B65D 83/24, B65D 83/384, B65D 83/14

(54) **VORRICHTUNG ZUM AUFNEHMEN EINER DRUCKDOSE FÜR EIN REINIGUNGSGERÄT UND REINIGUNGSGERÄT**

(30) Priorität: 20.02.2025 BE 202500018
(71) Anmelder: Miele & Cie. KG, 33332 Gütersloh (DE)
(72) Erfinder: Goldberg, Gregor, 48231 Warendorf (DE); Deppe, Fabian Lennart, 32107 Bad Salzuflen (DE)

(57) **Zusammenfassung**

Eine Vorrichtung (170) zum Aufnehmen einer Druckdose (155) für ein Reinigungsgerät (100) für medizinische Instrumente, wobei die Druckdose (155), einen Ventilbund, sowie ein Dosenventil zum Ausgeben eines Pflegemittels aufweist, umfasst einen Einsatz (200) mit einer Adaptionsstelle für die Druckdose (155), wobei der Einsatz (200) an der Adaptionsstelle eine Öffnung (225) zum Aufnehmen des Dosenventils und eine Führungskulisse aufweist, die an einer dosenseitigen Seite der Öffnung (225) angeordnet ist. Ferner umfasst die Vorrichtung einen Adaptionsring (210), der dazu ausgeformt ist, um das Dosenventil ringförmig zu umschließen. Dabei weist der Adaptionsring (210) eine Kante (310) zum Greifen unter dem Ventilbund, einen Hebel (235) zum Versetzen des Adaptionsrings (210) in eine Drehbewegung und eine Gegenkulisse auf, wobei die Gegenkulisse ausgebildet ist, um mit der Führungskulisse des Einsatzes (200) zusammenzuwirken, um den Adaptionsring (210) ansprechend auf die Drehbewegung in Richtung der Öffnung (225) zu bewegen.

## Beschreibung

Die Erfindung betrifft eine Vorrichtung zum Aufnehmen einer Druckdose für ein Reinigungsgerät und ein Reinigungsgerät für medizinische Instrumente.

Die EP 0 638 297 B1 beschreibt ein Verfahren und eine Vorrichtung zur hygienischen Aufbereitung von medizinischen, insbesondere zahnmedizinischen Instrumenten.

Der hier vorgestellte Ansatz stellt sich die Aufgabe, eine verbesserte Vorrichtung zum Aufnehmen einer Druckdose für ein Reinigungsgerät und ein verbessertes Reinigungsgerät für medizinische Instrumente zu schaffen.

Erfindungsgemäß wird diese Aufgabe durch eine Vorrichtung zum Aufnehmen einer Druckdose für ein Reinigungsgerät und ein Reinigungsgerät für medizinische Instrumente mit den Merkmalen der Hauptansprüche gelöst. Vorteilhafte Ausgestaltungen und Weiterbildungen der Erfindung ergeben sich aus den nachfolgenden Unteransprüchen.

Der mit der Erfindung erreichbare Vorteil besteht aus einer erhöhten Kompatibilität mit unterschiedlichen Pflegemitteldosen, unabhängig der Größe der Pflegemitteldose.

Eine Vorrichtung zum Aufnehmen einer Druckdose für ein Reinigungsgerät, wobei die Druckdose einen Ventilbund sowie ein Dosenventil zum Ausgeben eines Pflegemittels aufweist, weist folgende Merkmale auf:
einen Einsatz mit einer Adaptionsstelle für die Druckdose, wobei der Einsatz an der Adaptionsstelle eine Öffnung zum Aufnehmen des Dosenventils und eine Führungskulisse aufweist, die an einer dosenseitigen Seite der Öffnung angeordnet ist; und
einen Adaptionsring, der dazu ausgeformt ist, um das Dosenventil ringförmig zu umschließen, wobei der Adaptionsring eine Kante zum Greifen unter dem Ventilbund, einen Hebel zum Versetzen des Adaptionsrings in eine Drehbewegung und eine Gegenkulisse aufweist, wobei die Gegenkulisse ausgebildet ist, um mit der Führungskulisse des Einsatzes zusammenzuwirken, um den Adaptionsring ansprechend auf die Drehbewegung in Richtung der Öffnung zu bewegen.

Dabei kann das Reinigungsgerät gemäß einer Ausführungsform als ein Reinigungs- und Desinfektionsgerät ausgeformt sein, spezifischer kann das Reinigungsgerät ein Dentalpflegegerät sein. Das Reinigungsgerät kann somit zur Reinigung medizinischer Instrumente verwendet werden. Die Druckdose kann als eine Pflegemitteldose zum Bevorraten von Pflegemittel ausgeformt sein. Das Pflegemittel kann beispielsweise nach einer Reinigung medizinischer Instrumente verwendet werden, um die medizinischen Instrumente zu pflegen. Dabei kann das Pflegemittel in der Pflegemitteldose beispielsweise ein Öl, insbesondere Weißöl sein. Die Vorrichtung kann beispielsweise in einer Schublade, insbesondere an einer Rückseite einer Schublade in einem Reinigungsgerät angeordnet sein. Die Druckdose kann dabei als eine Pflegemitteldose ausgeformt sein, welche Pflegemittel für das Reinigungsgerät beinhaltet. Die Druckdose weist dabei wie übliche Druckdosen oben den Ventilbund und das Dosenventil auf. Der Ventilbund kann beispielsweise ein zylinderförmiger oder kegelförmiger Bereich der Druckdose zwischen dem Dosenventil und einem Hauptkörper der Druckdose angeordnet sein. Das Dosenventil kann dabei zu einer Ausgabe des Pflegemittels dienen. Der Einsatz der Vorrichtung kann eine Wanne sein, in welcher die Druckdose eingesetzt werden kann. Der Einsatz kann gemäß einer Ausführungsform aus Kunststoff oder Edelstahl ausgeformt sein. Die Adaptionsstelle kann beispielsweise dazu ausgeformt sein, um die Druckdose daran in Richtung der Öffnung des Einsatzes entlangzuführen. Dabei kann die Öffnung als eine Durchgangsöffnung ausgeformt sein. Die Führungskulisse kann beispielsweise als eine Schräge, Rampe oder Nut ausgeformt sein, in welcher die Gegenkulisse geführt werden kann. Die Gegenkulisse kann beispielsweise als eine Feder oder eine Nase ausgeformt sein. Die Gegenkulisse kann aus Führungsfortsätzen bestehen. Der Adaptionsring kann beispielsweise aus Kunststoff ausgeformt sein, um Produktionskosten zu sparen. Die Kante kann dabei ein in ein Inneres des Adaptionsrings zeigender Vorsprung sein. Der Hebel kann an einer Außenseite des Adaptionsring angeordnet sein.

Dabei kann der Einsatz an der Adaptionsstelle eine Dosenaufnahme aufweisen. Die Dosenaufnahme kann als eine Ablagefläche für die Druckdose ausgeformt sein. Die Dosenaufnahme kann dabei einteilig mit dem Einsatz ausgeformt sein. Die Dosenaufnahme kann ein Einsetzen der Druckdose in den Einsatz erleichtern.

Der Einsatz kann gemäß einer Ausführungsform als eine Wanne ausgeformt sein. Die Wanne kann eine Verjüngung aufweisen, welche an einem der Adaptionsstelle gegenüberliegenden Seite angeordnet ist, um ein dem Dosenventil abgewandtes Ende der Druckdose in dem Einsatz zu halten. Die Verjüngung kann dabei eine Unterstützung für die Druckdose darstellen, und somit verhindern, dass ein Ende der Druckdose lose in dem Einsatz gehalten wird.

Die Wanne kann im Bereich der Verjüngung zumindest eine Rippe aufweisen, um die Druckdose in dem Einsatz zu unterstützen. Die Wanne kann dabei beispielsweise zwei Rippen an gegenüberliegenden Seiten der Wanne aufweisen, um die Druckdose an mehreren Anlagepunkten zu unterstützen.

Die Vorrichtung kann einen Ausgleichsring aufweisen, welcher aufschiebbar auf die Druckdose ausgeformt ist, und dazu ausgeformt ist, um einen Abstand zwischen der Druckdose und der Verjüngung der Wanne auszufüllen, um die Druckdose fest an einer Stelle zu halten, wenn die Druckdose in den Einsatz eingeführt ist. Der Ausgleichsring kann beispielsweise aus Kunststoff ausgeformt sein, welcher eine leicht raue Oberfläche hat, um Rutschen zu vermeiden. Der Ausgleichsring kann dabei einen Abstand zwischen der Druckdose und den Wänden der Verjüngung ausfüllen.

Gemäß einer vorteilhaften Ausführungsform kann die Vorrichtung eine Übergabeeinheit aufweisen, welche in der Öffnung des Einsatzes angeordnet ist, um das Dosenventil abzudichten. Die Übergabeeinheit kann dabei beispielsweise als ein Einbauteil ausgeformt sein, welches sich einfach einsetzen lässt.

Die Übergabeeinheit kann einen Dichtring und zusätzlich oder alternativ einen Rückstromverhinderer und zusätzlich oder alternativ eine Ventilhülse und zusätzlich oder alternativ eine Distanzhülse umfassen. Der Dichtring und die Hülsen können zur Herstellung einer fluiddichten Verbindung zwischen der Druckdose und dem Einsatz verwendet werden. Durch den Rückstromverhinderer kann ein Zurücklaufen des Pflegemittels zu der Druckdose verhindert werden.

Der Adaptionsring kann gemäß einer Ausführungsform eine Aussparung aufweisen, durch welche das Ventil und der Ventilbund der Druckdose durchführbar ist, wenn der Adaptionsring auf die Druckdose aufgeschoben wird. Dabei kann die Aussparung beispielsweise eine umgekehrte T-Form aufweisen. Beispielsweise kann ein erster Teil der Aussparung breiter ausgeformt sein, um den Ventilbund durchführen zu können, und ein zweiter Teil der Aussparung kann dünner ausgeformt sein, um das Ventil durchführen zu können.

Die Kante des Adaptionsrings kann dabei an einem dosenseitigen Ende des Adaptionsrings angeordnet sein. Die Kante kann dabei ein nach Innen gerichteter Kragen sein, der unter den Ventilbund greift, um die Druckdose in dem Adaptionsring zu fixieren.

Die Gegenkulisse kann an einem der Kante gegenüberliegende Ende des Adaptionsrings angeordnet sein. Somit kann die Dose an zwei Stellen besser fixiert werden.

Dabei kann die Gegenkulisse zumindest eine Nase umfassen. Die Nase kann die Druckdose nach einer Drehbewegung des Adaptionsrings im Zusammenspiel mit der Führungskulisse in einer Endposition halten. Dadurch kann die Druckdose stabilisiert und eine sichere Abgabe des Pflegemittels gewährleistet werden.

Die Führungskulisse kann gemäß einer Ausführungsform eine Schräge zum Führen der Gegenkulisse in Richtung der Öffnung aufweisen. Durch die Schräge wird die von dem Adaptionsring gehaltene Druckdose bei der Drehung des Adaptionsrings entlang einer Längsachse der Druckdose bewegt.

Ferner wird ein Reinigungsgerät für medizinische Instrumente mit einer Ausführungsform der genannten Vorrichtung vorgestellt. Das Reinigungsgerät kann beispielsweise als ein Reinigungs- und Desinfektionsgerät ausgeformt sein, insbesondere als ein Dentalpflegegerät.

Das Reinigungsgerät kann einen Spülraum zum Aufnehmen der medizinischen Instrumente aufweisen. Optional kann das Reinigungsgerät zumindest einen in dem Spülraum angeordneten Sprüharm aufweisen. Ferner kann das Reinigungsgerät einen Medienverteiler mit Anschlüssen für die medizinischen Instrumente und eine Leitung zum Leiten des Pflegemittels von der Vorrichtung zu dem Medienverteiler aufweisen. Somit kann Pflegemittel aus der Vorrichtung dem Medienverteiler mittels einer Leitung zugeleitet werden. Der Spülraum und somit die medizinischen Instrumente können beispielsweise mit Spülmittel aus dem Sprüharm bespritzt werden. Dabei kann das Reinigungsgerät auch ein Umwälzsystem aufweisen, durch das das Spülmittel aus dem Spülraum zurück zu dem Sprüharm gefördert werden kann.

Auch wenn der beschriebene Ansatz anhand eines Haushaltgeräts beschrieben wird, kann der hier beschriebene Ansatz entsprechend im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Ausführungsbeispiels eines Reinigungsgeräts;
- Figur 2: eine Darstellung eines Ausführungsbeispiels einer Vorrichtung;
- Figur 3: eine Detailansicht eines Ausführungsbeispiels einer Vorrichtung;
- Figur 4: eine perspektivische Darstellung eines Ausführungsbeispiels einer Vorrichtung;
- Figur 5: eine Darstellung eines Ausführungsbeispiels einer Druckdose;
- Figur 6: eine weitere Darstellung eines Ausführungsbeispiels einer Druckdose;
- Figur 7: eine Darstellung eines Ausführungsbeispiels einer Vorrichtung;
- Figur 8: eine weitere Darstellung eines Ausführungsbeispiels einer Vorrichtung;
- Figur 9: eine weitere Darstellung eines Ausführungsbeispiels einer Vorrichtung;
- Figur 10: eine Detailansicht eines Ausführungsbeispiels einer Vorrichtung; und
- Figur 11: eine Schnittdarstellung eines Ausführungsbeispiels einer Druckdose.

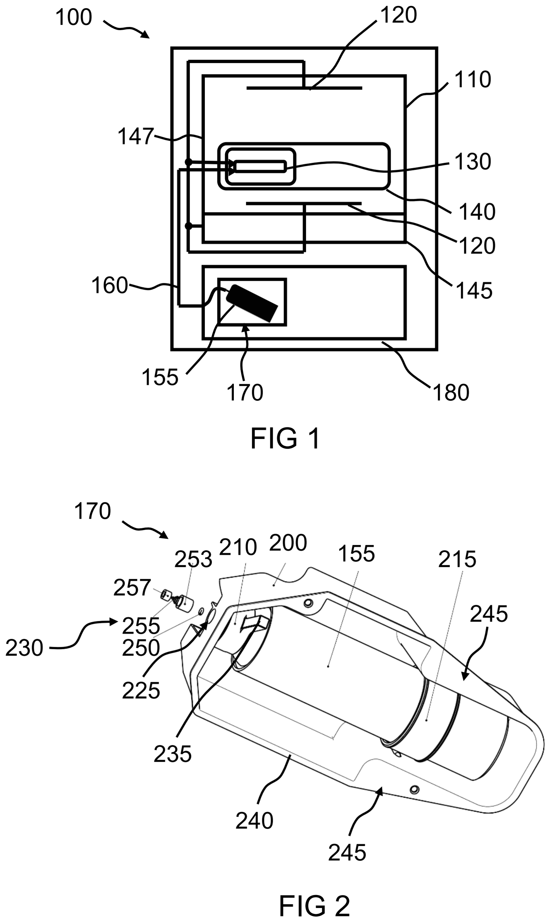

Figur 1 zeigt eine schematische Darstellung eines Ausführungsbeispiels eines Reinigungsgeräts 100, das beispielsweise zum Reinigen von medizinischen Instrumenten verwendet werden kann. Das Reinigungsgerät 100 weist einen Spülraum 110 auf, in den die medizinischen Instrumente zur Reinigung angeordnet werden können. Beispielsweise ist in dem Spülraum 110 zumindest ein Sprüharm 120 angeordnet, mit dem die medizinischen Instrumente mit einem Reinigungsmittel besprüht werden können. Dadurch können die medizinischen Instrumente äußerlich gereinigt werden. Optional ist in dem Spülraum 110 ein Medienverteiler 130 und ein Korbeinsatz 140 angeordnet. Die medizinischen Instrumente können von dem Korbeinsatz 140 gehalten werden und an den Medienverteiler 130 angeschlossen sein. Über den Medienverteiler 130 kann das Reinigungsmittel in ein Inneres der medizinischen Instrumente geleitet werden, um die medizinischen Instrumente von innen zu reinigen. Das Reinigungsmittel wird gemäß einem Ausführungsbeispiel am Boden des Spülraums 110 aufgefangen und während eines Reinigungsprozesses unter Verwendung eines Umwälzsystems 145 und Reinigungsleitungen 147 erneut zu dem zumindest einem Sprüharm 120 und dem Medienverteiler 130 geführt.

Zum Pflegen der medizinischen Instrumente, beispielsweise zum Ende eines Reinigungsprozesses wird ein Pflegemittel verwendet, das in einer Druckdose 155 bevorratet wird. Gemäß einem Ausführungsbeispiel wird das Pflegemittel aus der Druckdose 155 über eine Leitung 160 zu dem Medienverteiler 130 geleitet. Über den Medienverteiler 130 wird das Pflegemittel gemäß einem Ausführungsbeispiel in ein Inneres der medizinischen Instrumente geleitet.

Die Druckdose 155 ist von einer Vorrichtung 170 des Reinigungsgeräts 100 aufgenommen, welche beispielsweise in einer Pflege- und Drucklufteinheit 180 des Reinigungsgeräts 100 angeordnet ist.

Gemäß einem Ausführungsbeispiel ist das Reinigungsgerät 100 ein Reinigungs- und Desinfektionsgerät. Gereinigt werden Utensilien, beispielsweise verschiedene medizinische Instrumente, erst mit dem Reinigungsmittel, und anschließend werden diese optional mit Wasserdampf desinfiziert. Das von dem Umwälzsystem 145 geförderte Reinigungsmittel stellt dabei ein Fluid dar, welches auch als Reinigungsflotte bezeichnet wird.

Dabei ist das Reinigungsgerät 100 außerdem dazu ausgebildet, um die verschiedenen medizinische Instrumente oder auch andere Utensilien nach einem Reinigen und Desinfizieren zu Pflegen. Dabei wird das Pflegemittel, beispielsweise ein Pflegeöl, unter Verwendung der Vorrichtung 170 in der Pflege- und Drucklufteinheit 180 aus der Druckdose 155 entnommen und zu den zu pflegenden Utensilien geleitet. Das Gesamtsystem ist dabei in der Lage, medizinische Instrumente oder Ähnliches möglichst gut zu reinigen und instand zu halten.

Die Pflege- und Drucklufteinheit 180 ist gemäß dem hier gezeigten Ausführungsbeispiel als eine Schublade ausgeformt. Die Vorrichtung 170 ist beispielsweise als eine Pflegemittelaufnahme ausgeformt, in welcher die Druckdose 155 gelagert werden kann, welche das Pflegemittel beinhaltet.

Die Vorrichtung 170 nimmt dabei die Druckdose 155 für das Reinigungsgerät 100 auf. Die Druckdose 155 weist einen Ventilbund sowie ein Dosenventil zum Ausgeben des bevorrateten und unter Druck stehenden Pflegemittel auf. Die Vorrichtung 170 ist dabei beispielsweise als eine einheitliche Schnittstelle zur Adaption unterschiedlicher Druckdosen in dem Reinigungsgerät 100, beispielsweise in Form eines thermischen Desinfektors, ausgeformt.

Die Vorrichtung 170 umfasst dazu einen Einsatz mit einer Adaptionsstelle für die Druckdose 155, wobei der Einsatz an der Adaptionsstelle eine Öffnung zum Aufnehmen des Dosenventils aufweist. Ferner weist der Einsatz eine Führungskulisse auf, die an einer dosenseitigen Seite der Öffnung angeordnet ist. Die Führungskulisse ist dabei ausgeformt, um mit einer Gegenkulisse zusammenzuwirken, die an einem die Druckdose 155 haltenden Adaptionsring angeordnet ist. Der Adaptionsring weist dazu eine Kante zum Greifen unter den Ventilbund der Druckdose 155 sowie einen Hebel zum Versetzen des Adaptionsrings in eine Drehbewegung auf. Durch die Drehbewegung können die Gegenkulisse und die Führungskulisse des Einsatzes zusammenzuwirken, um den Adaptionsring und damit die von dem Adaptionsring gehaltene Druckdose 155 in Richtung der Öffnung des Einsatzes zu bewegen.

Es wird ein Aufbau und eine Integration eines Systems zur Aufnahme von Pflegemittelgebinden für medizinische Instrumente, beispielsweise für Übertragungsinstrumente in Form von Turbinen oder Hand- und Winkelstücken, in dem Reinigungsgerät 100, auch als Reinigungs- und Desinfektionsgerät, kurz RDG, vorgestellt. Ziel ist ein der automatisierten Instrumentenaufbereitung nachgelagerter und ebenfalls automatisierter Pflegeprozess innerhalb des Reinigungsgeräts 100.

Durch das Reinigungsgerät 100 mit der Vorrichtung 170 wird ein Einbringen von Pflegeöl zur Schmierung der Lagerungen und Getriebe innerhalb der Instrumente, nicht mehr manuell, sondern automatisch nach der Aufbereitung im Reinigungsgerät 100 und vor der Sterilisation ausgeführt. Hierfür werden an sich bekannte Druckdosen in Form von Sprühdosen mit einem entsprechenden Adapter eingesetzt, da sie sehr einfach anzuwenden sind und für eine zuverlässig eingebrachte Mindestmenge an Pflegeöl in die Instrumente sorgen.

Gemäß dem Stand der Technik werden die die Instrumente für den Pflegeschritt in einen separaten Pflegeautomaten eingesetzt.

Um den Gesamtprozess effizienter zu gestalten und einen Mehrwert für den Anwender zu erzeugen, soll dieser Prozessschritt nach der Aufbereitung automatisch und anwählbar innerhalb des Reinigungsgeräts 100 selbst erfolgen und somit die Entnahme der Instrumente für den Pflegeschritt unnötig machen.

Die maschinelle Aufbereitung der Übertragungsinstrumente einschließlich ihrer Pflege, setzt die Möglichkeit der Aufnahme der Druckdose 155 als ein Pflegegebinde innerhalb des Reinigungsgeräts 100 voraus. Die Druckdose 155 ist vergleichbar mit einer Standardsprühdose mit manuell betätigbarem Druckventil.

Das Öl ist hier gemäß einem Ausführungsbeispiel ein medizinisches Weißöl. Durch die Vorrichtung 170 ist es möglich unterschiedliche Öldosen zu adaptieren, unabhängig davon, welche vom Hersteller des Pflegegerätes empfohlen wird. Dadurch ist der Anwender welcher einen Pflegeautomaten, oder einen Aufbereitungsautomaten mit Pflegefunktion nutzt, nicht auf das Angebot des jeweiligen Geräteherstellers beschränkt.

Um dem Kunden die Möglichkeit zu geben, Öldosen aller Anbieter in einem Dental-RDG zu verwenden (Bevorratung und Freigabe des Mediums in das Pflegesystem) und somit eine Marktoffenheit bezüglich dieser Gebinde zu generieren, wird ein Schnittstellensystem beschrieben, welches eine prozess- und anwendungssichere universelle Aufnahme aller am Markt befindlichen Dosenausprägungen gewährleistet.

Das Reinigungsgerät 100 reinigt Dentalinstrumente chemisch und spült diese anschließend mit Pflegeöl durch, bzw. reinigt, ölt und sterilisiert die Instrumente anschließend. Die Prozesse finden anteilig mit Frischwasser innerhalb der jeweiligen Geräte statt. Der Anwender wählt zuvor aus, welche Kombination aus Reinigungs-, Desinfektions- und Pflegezyklus angewendet werden soll.

Das Reinigungsgerät 100 hat dabei einen Frischwasserprozess zur thermischen Innen- und Außenaufbereitung der Instrumente, welcher eine sehr hohe Hygieneleistung aufweist. Durch das beschriebene Reinigungsgerät 100 wird nun eine anschließende Ölung der Instrumente innerhalb des Reinigungsgeräts 100 möglich sein, um den sehr guten Aufbereitungsprozess mit diesem Anwendervorteil zu kombinieren. Für die Pflege von dentalen Übertragungsinstrumenten wird dabei auch ein Pflegemittel benötigt. Dieses soll in Form der beschriebenen Druckdose 155, auch als Pflegemitteldose bezeichnet, im Geräteträger des jeweiligen Reinigungsgeräts 100 prozess- und anwendungssicher vom Kunden eingesetzt werden können.

Dabei sollen möglichst alle Gebindegeometrien von vorhandenen Sprühmitteldosen berücksichtigt werden und die Adaption ihrer Pflegemitteldosen möglich sein. Um diese Bandbreite an Pflegemitteldosen abzudecken, ist eine universelle Schnittstelle mit einer Aufnahmemöglichkeit vorgesehen, für welche die verschiedenen Durchmesser und Längen der Dosen keine Einschränkung darstellt. Der hier beschriebene Ansatz beschreibt eine konstruktive Umsetzung eines Adaptionssystems mit herstellerunabhängiger Schnittstelle, um Pflegemitteldosen in einem Dental-RDG mit integrierter Pflegefunktion adaptieren zu können.

Figur 2 zeigt eine schematische Darstellung eines Ausführungsbeispiels einer Vorrichtung 170 zum Aufnehmen einer Druckdose 155. Die Vorrichtung 170 ist gemäß einem Ausführungsbeispiel wie die in Figur 1 beschriebene Vorrichtung ausgeformt. Die Vorrichtung 170 umfasst den Einsatz 200, eine Druckdose 155, auf die ein Adaptionsring 210 und optional ein Ausgleichsring 215 aufgesetzt ist. Das Dosenventil der Druckdose 155 ist in einer Öffnung 225 des Einsatzes 200 angeordnet und wird von einer Übergabeeinheit 230 abgedichtet.

Die Druckdose 155 beinhaltet hier beispielshaft ein Reinigungsöl, beispielsweise ein Weißöl. Vor dem Einsetzen der Druckdose 155 in den Einsatz 200, wird der Adaptionsring 210 auf den Ventilbund aufgeschoben. Der Adaptionsring 210 hat dabei einen Hebel 235, um die Druckdose 155 in eine geschlossene Position zu drehen, wenn diese in dem Einsatz 200 eingesetzt ist.

Der Einsatz 200 ist beispielhaft als eine Wanne ausgeformt. Optional weist eine Wand 240 des Einsatzes 200 eine Verjüngung 245 auf. Die Verjüngung 245 ist dazu da, um die Druckdose 155 an einer weiteren Stelle, hier an einem dem Dosenventil abgewandten Ende eines Behälters der Druckdose 155, zu unterstützen. Der Ausgleichsring 215 umschließt dabei den Behälter, um einen Abstand zwischen der Verjüngung 245 und der Druckdose 155 auszufüllen. Der Ausgleichsring 215 wird dann eingesetzt, wenn die Druckdose 155 zu klein für den Einsatz 200 ist.

Die Übergabeeinheit 230 zum Abdichten der Druckdose 155 an der Öffnung 225, besteht beispielhaft aus einem Dichtring 250, einer Ventilhülse 253, einem Rückstromverhinderer 255 und einer Distanzhülse 257. Der Dichtring 250 ist dabei beispielsweise ein O-Ring mit den Abmessungen 5x2,5. Die Distanzhülse 257 kann dabei Maße von 7x4,1x5 aufweisen. An der Öffnung 225 ist gemäß einem Ausführungsbeispiel eine Steckverschraubung angebracht, welche in der Figur 2 nicht abgebildet ist

Mit der hier vorgestellten Vorrichtung 170 können Pflegemitteldosen unterschiedlicher Hersteller verwendet werden. Die Durchmesserbegrenzung einer Einschuböffnung entfällt, da beispielsweise von der größtmöglichen verfügbaren Pflegemitteldose ausgegangen wird. Weichen die Dosen von der vorgegebenen Geometrie ab ist eine sichere Adaption durch den Adaptionsring 210 und den Ausgleichsring 215 möglich. Die Düse der Druckdose 155 kann geöffnet werden, auch wenn die Druckdose 155 kurz ist. Auch eine Breite der Druckdose 155 ist aufgrund des Ausgleichsring 215 kein Problem mehr. Somit ist auch keine Festlegung auf ein bestimmtes Pflegespray durch die Abmaße der Dosen oder eine spezielle Aufnahmegeometrie am Ventilende der Dose zu diesem Zweck nötig.

Vorteilhafterweise ist der konstruktive Aufwand und die Komplexität der Baugruppe für die Adaption unterschiedlicher Gebindegrößen sehr gering. Wenn der Anwender ein alternatives Pflegemittel verwenden möchte, muss er nicht mehr auf den manuelle Pflegeprozess ausweichen und hat keine damit verbunden Nachteile, wie einen erhöhten Prozessaufwand, einen Zeitverlust innerhalb der Prozesskette oder das Risiko einer Rekontamination.

Da das System nach dem Einsetzen der Pflegemitteldose 155 mit dem in der Pflegemitteldose 155 befindlichen Druckniveau beaufschlagt wird, stellt sich dieser Druck auch zwischen dem Dosenventil und der Anschlussstelle für den Versorgungsschlauch der geräteinternen Pflegemittelversorgung ein.

Der optionale Rückstromverhinderer 255 unterbindet den möglichen Rückfluss und somit das Austreten des sich zwischen Dosenventil und Schlauchanschluss befindlichen Pflegemittels bei der Entnahme der Pflegemitteldose 155, da beim Entlasten des Dichtringes 250 das Druckniveau in Richtung der Pflegemitteldose 155 abfällt. Die Schnittstelle zum Reinigungsgerät 100 wird beispielsweise mittels einer Steckverschraubung umgesetzt. An dieser kann ein Schlauch mit dem benötigten Durchmesser angeschlossen werden.

Da die zur Adaption der Pflegemitteldose 155 benötigte Kraft ausschließlich über die Haltekräfte zwischen Adaptionsring 210 und Ventilbund übertragen werden, entfallen jegliche geometrischen Restriktionen bezüglich der Länge der Pflegemitteldose 155.

Um der Varianz bezüglich der Dosendurchmesser zu begegnen, ist der Einsatz 200 beispielsweise so konstruiert, dass er die größten am Markt befindlichen Pflegemitteldosen aufnehmen kann. Zur Abstützung, leichten Fixierung und Führung dieses Dosendurchmessers sind beispielsweise flexible Laschen seitlich in der Außenkontur des Einsatzes 200 intergiert. Für kleinere Dosendurchmesser wird der Ausgleichsring 215 auf die Pflegemitteldose 155 geschoben, welcher den abweichenden Durchmesser ausgleicht und somit ebenfalls von optionalen Führungslaschen im Einsatz 200 aufgenommen wird.

Der Mehrwert für den Anwender besteht in der freien Wahl des Pflegemittelgebindes ohne etwaige geometrische Beschränkungen. Zudem erfolgt durch Integration des Pflegeprozesses in das Reinigungsgerät 100 eine Prozessoptimierung bezüglich der Dauer des Gesamtprozesses inklusive der Instrumentenentnahme zur manuellen Pflege, sowie eine Risikoreduktion bezüglich einer möglichen Rekontamination.

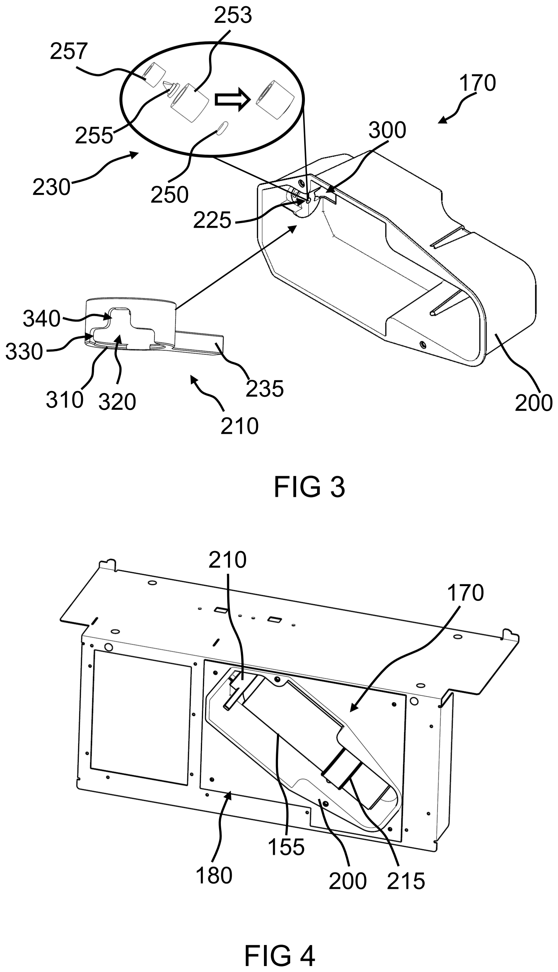

Figur 3 zeigt eine Detailansicht eines Ausführungsbeispiels einer Vorrichtung 170. Die Vorrichtung 170 ist dabei gemäß einem Ausführungsbeispiel wie die in Figur 1 beschriebene Vorrichtung ausgeformt. Es ist die Übergabeeinheit 230, der Adaptionsring 210 und eine Dosenaufnahme 300 in dem Einsatz 200 abgebildet.

Hier werden verschiedene Teilsysteme des Einsatzes 200 beschrieben. Die Übergabeeinheit 230 ist dabei wie die in Figur 2 beschrieben Übergabeeinheit 230 ausgeformt, und hier im Detail angezeigt. Dabei ist die Übergabeeinheit 230 an der Öffnung 225 angeordnet. Ebenfalls an der Öffnung 225, hierbei in Richtung einer dosenseitigen Seite, ist die Dosenaufnahme 300 angeordnet. Die Dosenaufnahme 300 ist hierbei als eine Ablage für die Druckdose ausgeformt.

Der Adaptionsring 210 wird ebenfalls in einer genaueren Ansicht dargestellt. Dabei hat der Adaptionsring 210 den Hebel 235, eine Kante 310 und eine Gegenkulisse. Die Kante 310 ist auf einer Innenseite des Adaptionsrings 210 angeordnet. Die Kante 310 ist dazu da, um unter einen Ventilbund der Druckdose zu greifen, und diese somit fest mit dem Adaptionsring 210 zu verbinden. Dafür weist der Adaptionsring 210 eine Aussparung 320 auf, welche dazu ausgeformt ist, um den Ventilbund und das Dosenventil durchzuführen. Dabei ist ein erster Teil 330 der Aussprung breiter, um das Ventilbund durchzuführen, und ein zweiter Teil 340 der Aussparung 320 ist dünner, um das Dosenventil durchzuführen. Die Aussparung 320 hat somit beispielsweise die Form eines umgekehrten "T".

Der hier beschriebene Ansatz stellt die Umsetzung einer einheitlichen Schnittstelle zur herstellerunabhängigen Nutzung von Druckdosengebinden dar. Dabei wird im Moment der Adaption das in der Druckdose befindliche Pflegemittel freigegeben und kann durch nachgeschaltete Aktoren, wie zum Beispiel Ventile, in den geräteinternen Pflegeprozess übergeben werden. Voraussetzung zur Umsetzung dieses Ansatzes ist der geometrisch bei allen Sprühdosen identische und dort fest verpresste Ventileinsatz, welcher zur Aufnahme des Gebindes mittels des aufschiebbaren Adaptionsringes 210 dient. Um eine Adaption aller gängigen Pflegemitteldosen zu gewährleisten, gliedert sich das hier beschriebene System grundsätzlich in die Teilsysteme des Einsatzes 200, der Dosenaufnahme 300, des Adaptionsringes 210 und optional der Übergabeeinheit 230 mit integrierter Abdichtung durch einen Dichtring 250 und einer Rückflussverhinderung.

Bei geöffnetem Ventil drückt die Feder des Dosenventils gemäß einem Ausführungsbeispiel einen Stiftanschluss in einen dafür vorgesehenen Dichtsitz. Dieser besteht aus dem Dichtring 250, welcher auf einem Absatz innerhalb der Übergabeeinheit 230 aufliegt und einer Ventilhülse 253. Diese Ventilhülse 253 fixiert den Dichtring 250. Direkt im Anschluss des Dichtringes 250 ist ein Rückstromverhinderer 255 in die Übergabeeinheit 230 integriert, hier in der Ausprägung eines Duckbill-Ventils. Das Duckbill Ventil ist dabei beispielsweise ein Duckbill D 3,9. Umgeben ist der Rückstromverhinderer 255 dabei von der Distanzhülse 257.

Figur 4 zeigt eine perspektivische Darstellung eines Ausführungsbeispiels einer Vorrichtung 170. Die Vorrichtung 170 ist dabei gemäß einem Ausführungsbeispiel wie die in Figur 1 beschriebene Vorrichtung ausgeformt. Der Einsatz 200 ist hierbei in der Pflege- und Drucklufteinheit 180 des Reinigungsgeräts 100 verbaut. Der Einsatz 200 nimmt die Druckdose 155 mit dem Adaptionsring 210 und dem Ausgleichsring 215 auf.

Der Einsatz 200 ist beispielsweise diagonal in der Pflege- und Drucklufteinheit 180 angeordnet. Die Pflege- und Drucklufteinheit 180 ist dabei gemäß einem Ausführungsbeispiel in einer Schublade, genauer in einer Schubladenrückwand angeordnet. Die Einbausituation in Schubladenrückwand ermöglicht es einem Nutzer, die Druckdose 155 leicht zu wechseln, und im Falle eines Defekts Reparaturen zu erleichtern.

Alle Teilsysteme sind innerhalb des Bauraumes des Einsatzes 200 angeordnet. Dieser ist in der Schubladenrückwand des Geräteträgers des jeweiligen Reinigungsgeräts integriert und so, im geöffneten Zustand der Schublade, vom Anwender erreichbar.

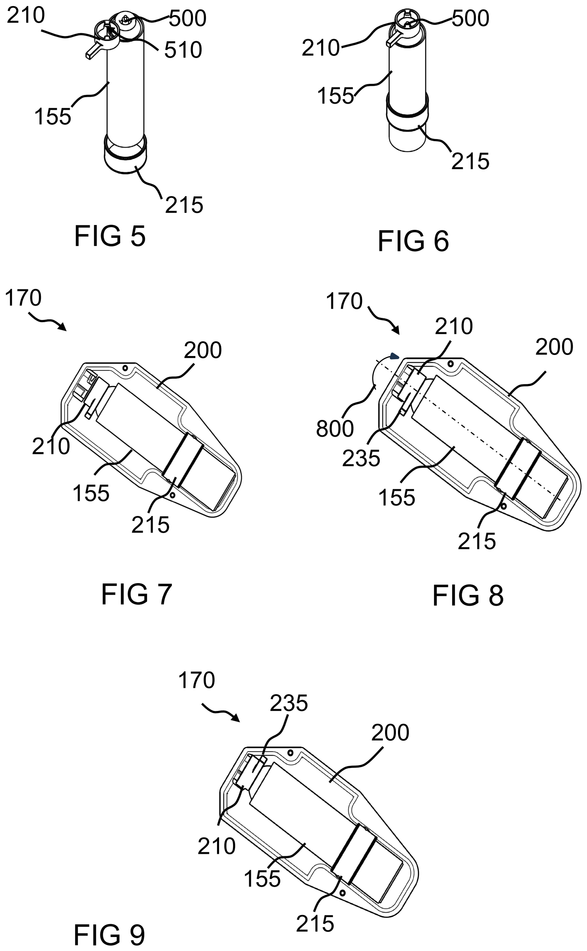

Figur 5 zeigt eine Darstellung eines Ausführungsbeispiels einer Druckdose 155. Die Druckdose 155 ist dabei gemäß einem Ausführungsbeispiel wie die in Figur 2 beschriebene Druckdose ausgeformt. Die Druckdose 155 ist hier nicht in dem Einsatz aufgenommen.

Die Druckdose 155 ist hier weder in dem Adaptionsring 210 noch in dem Ausgleichsring 215 angeordnet. Der Adaptionsring 210 kann dabei von der Seite auf ein Dosenventil 500 aufgeschoben werden. Der Ausgleichsring 215 kann dabei von einem dem Dosenventil 500 gegenüberliegenden Ende der Druckdose 155 aufgezogen werden. In einem Anwendungsablauf ist der hier gezeigte Schritt der erste Schritt beim Montieren der Druckdose 155.

Der Adaptionsring 210 wird dabei über die Aussparung auf die Druckdose 155 aufgeschoben. An einer der Druckdose 155 gegenüberliegenden Seite des Adaptionsrings 210 ist dabei eine Gegenkulisse 510 angeordnet. Die Gegenkulisse 510 ist dazu da, um in einer Führungskulisse des Einsatzes geführt zu werden. Dabei ist die Gegenkulisse 510 beispielsweise als eine Nase ausgeformt.

Gemäß einem Ausführungsbeispiel wird zuerst der Adaptionsring 210 seitlich über den Bund des Dosenventils 500 der Druckdose 155 aufgeschoben. Dieser Bund ist fester Bestandteil der Druckdose 155 oder des Dosenventils 500. Die Fertigungsmethode des Ventils, sein funktionaler sowie sein geometrischer Aufbau sind bei den gängigen Pflegemitteldosen immer identisch. Der Adaptionsring 210 greift beim Aufschieben mit einer erhabenen Kante unter den Ventilbund der Druckdose 155 und umschließt ihn im aufgeschobenen Zustand nahezu vollständig. An dem Adaptionsring 210 befinden sich optional zudem Fortsätze der Gegenkulisse 510 zur späteren Verriegelung der Dose, welche sich nach Anbringung des Adaptionsringes 210 oberhalb der Ventilöffnung befinden. Der Adaptionsring 210 weist zudem eine Kontur auf, um ihn manuell auf dem Ventilbund um die Dosenachse verdrehen zu können.

Figur 6 zeigt eine weitere Darstellung eines Ausführungsbeispiels einer Druckdose 155. Die Druckdose 155 ist dabei gemäß einem Ausführungsbeispiel wie die in Figur 5 beschriebene Druckdose ausgeformt. Die Druckdose 155 ist auch hier nicht in dem Einsatz aufgenommen.

Der Adaptionsring 210 ist hierbei an dem Dosenventil 500 angebracht und der Ausgleichsring 215 ist ebenfalls auf die Druckdose 155 aufgeschoben.

Figur 7 zeigt eine Darstellung eines Ausführungsbeispiels einer Vorrichtung 170. Die Vorrichtung 170 ist dabei gemäß einem Ausführungsbeispiel wie die in Figur 1 beschriebene Vorrichtung ausgeformt. Hier ist die Druckdose 155 mit dem Adaptionsring 210 und dem Ausgleichsring 215 in dem Einsatz 200 aufgenommen.

In dem Anwendungsablauf wird nach dem in den Figuren 5 und 6 gezeigten Aufsetzen des Adaptionsrings 210 und des Ausgleichsrings 215 die Druckdose 155 in den Einsatz 200 eingelegt. Dabei ist die Druckdose 155 von der Dosenaufnahme aufgenommen, allerdings noch nicht mit der Übergabeeinheit gekoppelt.

Die so mit dem Adaptionsring 210 kraftschlüssig verbundene Pflegemitteldose wird dazu in den auch als Pflegemitteleinsatz bezeichneten Einsatz 200 eingelegt und optional durch im Einsatz 200 eingebrachte flexible Laschen seitlich geführt.

Figur 8 zeigt eine weitere Darstellung eines Ausführungsbeispiels einer Vorrichtung 170. Die Vorrichtung 170 ist dabei gemäß einem Ausführungsbeispiel wie die in Figur 7 beschriebene Vorrichtung ausgeformt. Hier ist die Druckdose 155 mit dem Adaptionsring 210 und dem Ausgleichsring 215 in dem Einsatz 200 aufgenommen.

Dabei ist eine Drehrichtung als ein Pfeil 800 dargestellt. Der Pfeil 800 zeigt dabei die Richtung, in welche der Hebel 235 des Adaptionsring 210 betätigt werden soll, um eine Kupplung der Druckdose 155 und der Übergabeeinheit zu erzielen. Durch das Betätigen des Hebels 235, wird die Gegenkulisse des Adaptionsrings 210 entlang einer Schräge der Kulisse geführt. Dadurch wird die Druckdose 155 in Richtung der Öffnung bewegt, und das Dosenventil geöffnet.

Figur 9 zeigt eine weitere Darstellung eines Ausführungsbeispiels einer Vorrichtung 170. Die Vorrichtung 170 ist dabei gemäß einem Ausführungsbeispiel wie die in Figur 8 beschriebene Vorrichtung ausgeformt. Hier ist die Druckdose 155 mit dem Adaptionsring 210 und dem Ausgleichsring 215 in dem Einsatz 200 aufgenommen.

Die Druckdose 155 hat hierbei den in Figur 8 beschriebenen Anwendungsablauf durchlaufen, und ist nach der Drehung des Adaptionsrings 210 fest in dem Einsatz 200 eingespannt. Der Hebel 235 zeigt nun in eine andere Richtung als den in den Figuren 7 oder 8 beschriebenen Montagezuständen. Sowohl durch den Adaptionsring 210 als auch den Ausgleichsring 215 wird die Druckdose 155 somit fest in dem Einsatz 200 gehalten.

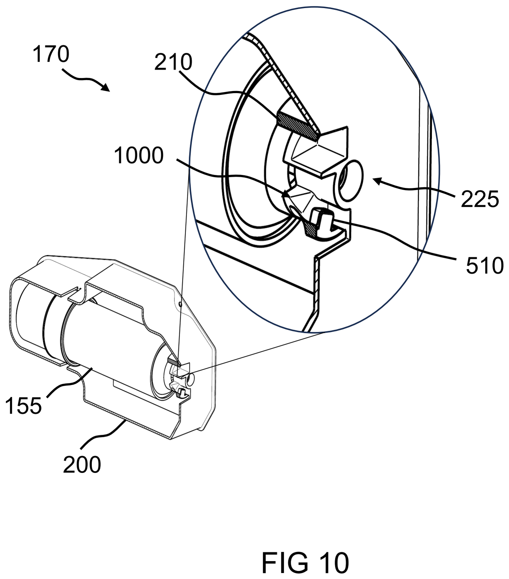

Figur 10 zeigt eine Detailansicht eines Ausführungsbeispiels einer Vorrichtung 170. Die Vorrichtung 170 ist dabei gemäß einem Ausführungsbeispiel wie die in Figur 1 beschriebene Vorrichtung ausgeformt. Genauer wird eine Detailansicht der Führungskulisse 1000 des Einsatzes 200 gezeigt.

Die Führungskulisse 1000 ist an der Öffnung 225 des Einsatzes 200 angeordnet und dazu ausgeformt, um zusammen mit der Gegenkulisse 510 des Adaptionsrings 210 die Druckdose 155 zu der Öffnung 225 zu bewegen und an der Übergabeeinheit zu koppeln.

Dabei umfasst die Führungskulisse 1000 eine Schräge, welche dafür sorgt, dass sich der Adaptionsring 210 bei einer Drehung in Richtung der Öffnung 225 bewegt und dabei die Druckdose 155 mitnimmt. Die Gegenkulisse 510 ist beispielhaft als eine Nase ausgeformt, die bei der Drehung des Adaptionsrings 210 entlang der Führungskulisse 1000 entlanggeführt wird.

Durch die Drehbewegung und die in der Adaptionsstelle eingebrachte Führungskulisse 1000, in welcher sich beispielsweise Führungsfortsätze der Gegenkulisse 510 bewegen, wird der Adaptionsring 210 mitsamt der Druckdose 155 linear bis zur vollständigen Öffnung des doseninternen Ventils bewegt. Der Drehwinkel des Adaptionsringes 210 ist dabei so bemessen, dass der lineare Weg der Druckdose 155 immer zu einer vollständigen Öffnung des Ventils führt.

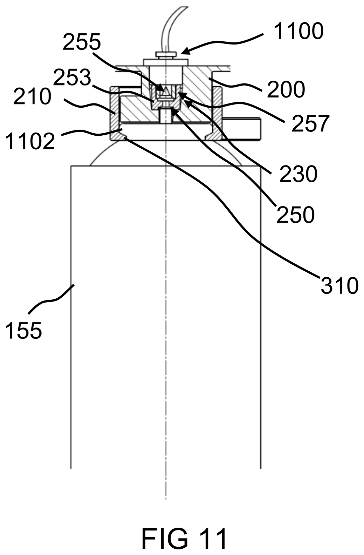

Figur 11 zeigt eine Schnittdarstellung eines Ausführungsbeispiels einer Druckdose 155. Die Druckdose 155 ist dabei gemäß einem Ausführungsbeispiel wie die in Figur 2 beschriebene Druckdose ausgeformt. Die Druckdose 155 ist hierbei in dem Adaptionsring 210 und in der Übergabeeinheit 230 angeordnet. Dabei wird der Ventilbund 1102 der Druckdose 155 von der Kante 310 des Adaptionsrings 210 gehalten.

Hier wird ein Schnittstellenmechanismus gezeigt, mit welchem die Druckdose 155 in dem Einsatz 200 eingesetzt ist. Der Schnittstellenmechanismus setzt sich hierbei zusammen aus dem Einsatz 200, dem Adaptionsring 210, der Übergabeeinheit 230, mit der Ventilhülse 253, der Distanzhülse 257, dem Rückstromverhinderer 255 und dem Dichtring 250, sowie aus einer Steckverschraubung 1100.

Dabei umschließt der Adaptionsring 210 die Führungskulisse des Einsatzes 200. Das Dosenventil wird der Reihenfolge nach von dem Dichtring 250, der Ventilhülse 253 und dann dem Rückstromverhinderer 255 in der Distanzhülse 257 abgedichtet. Das Dosenventil ist in dem gezeigten Endmontagezustand der Druckdose 155 dauerhaft geöffnet. Gemäß einem Ausführungsbeispiel weist die Steckverschraubung 1100 einen weiteren Dichtring auf. Die Steckverschraubung 1110 ist dabei beispielsweise dazu gedacht, um eine Leitung oder einen Schlauch aufzunehmen, um das Reinigungsmittel aus der Druckdose 155 abzuleiten, wie es beispielsweise anhand von Figur 1 beschrieben ist.

## Patentansprüche

1. Vorrichtung (170) zum Aufnehmen einer Druckdose (155) für ein Reinigungsgerät (100), wobei die Druckdose (155), einen Ventilbund (1102), sowie ein Dosenventil (500) zum Ausgeben eines Pflegemittels aufweist, und wobei die Vorrichtung (170) folgende Merkmale aufweist:
einen Einsatz (200) mit einer Adaptionsstelle für die Druckdose (155), wobei der Einsatz (200) an der Adaptionsstelle eine Öffnung (225) zum Aufnehmen des Dosenventils (500) und eine Führungskulisse (1000) aufweist, die an einer dosenseitigen Seite der Öffnung (225) angeordnet ist; und
einen Adaptionsring (210), der dazu ausgeformt ist, um das Dosenventil (500) ringförmig zu umschließen, wobei der Adaptionsring (210) eine Kante (310) zum Greifen unter dem Ventilbund (1102), einen Hebel (235) zum Versetzen des Adaptionsrings (210) in eine Drehbewegung, und eine Gegenkulisse (510) aufweist, wobei die Gegenkulisse (510) ausgebildet ist, um mit der Führungskulisse (1000) des Einsatzes (200) zusammenzuwirken, um den Adaptionsring (210) ansprechend auf die Drehbewegung in Richtung der Öffnung (225) zu bewegen.

2. Vorrichtung (170) gemäß Anspruch 1, wobei der Einsatz (200) an der Adaptionsstelle eine Dosenaufnahme (300) aufweist, welche als eine Ablagefläche für die Druckdose (155) ausgeformt ist.

3. Vorrichtung (170) gemäß einem der vorangegangenen Ansprüche, wobei der Einsatz (200) als eine Wanne ausgeformt ist, wobei die Wanne eine Verjüngung (245) aufweist, welche an einem der Adaptionsstelle gegenüberliegenden Seite angeordnet ist, um ein dem Dosenventil (500) abgewandtes Ende der Druckdose (155) in dem Einsatz (200) zu halten.

4. Vorrichtung (170) gemäß Anspruch 3, wobei die Wanne im Bereich der Verjüngung (245) zumindest eine Rippe aufweist, um die Druckdose (155) in dem Einsatz (200) zu unterstützen.

5. Vorrichtung (170) gemäß einem der Ansprüche 3 oder 4, mit einem Ausgleichsring (215) welcher aufschiebbar auf die Druckdose (155) ausgeformt ist, und dazu ausgeformt ist, um einen Abstand zwischen der Druckdose (155) und der Verjüngung (245) der Wanne auszufüllen, um die Druckdose (155) fest an einer Stelle zu halten, wenn die Druckdose (155) in dem Einsatz (200) eingeführt ist.

6. Vorrichtung (170) gemäß einem der vorangegangenen Ansprüche, mit einer Übergabeeinheit (230), welche in der Öffnung (225) des Einsatzes (200) angeordnet ist, um das Dosenventil (500) abzudichten.

7. Vorrichtung (170) gemäß Anspruch 6, wobei die Übergabeeinheit (230) einen Dichtring (250) und/oder einen Rückstromverhinderer (255) und/oder eine Ventilhülse (253) und/oder eine Distanzhülse (257) umfasst.

8. Vorrichtung (170) gemäß einem der vorangegangenen Ansprüche, wobei der Adaptionsring (210) eine Aussparung (320) aufweist, durch welche das Dosenventil (500) und der Ventilbund (1102) der Druckdose (155) durchführbar ist, wenn der Adaptionsring (210) auf die Druckdose (155) aufgeschoben wird.

9. Vorrichtung (170) gemäß einem der vorangegangenen Ansprüche, wobei die Kante (310) des Adaptionsrings (210) an einem dosenseitigen Ende des Adaptionsrings (210) angeordnet ist, und wobei die Gegenkulisse (510) an einem der Kante (310) gegenüberliegende Ende des Adaptionsrings (210) angeordnet ist.

10. Vorrichtung (170) gemäß einem der vorangegangenen Ansprüche, wobei die Gegenkulisse (510) zumindest eine Nase umfasst.

11. Vorrichtung (170) gemäß einem der vorangegangenen Ansprüche, wobei die Führungskulisse (1000) eine Schräge zum Führen der Gegenkulisse (510) in Richtung der Öffnung (225) aufweist.

12. Reinigungsgerät (100) für medizinische Instrumente mit einer Vorrichtung (170) gemäß den Ansprüchen 1 bis 11.

13. Reinigungsgerät (100) gemäß Anspruch 12, mit einem Spülraum (110) zum Aufnehmen der medizinischen Instrumente, zumindest einem in dem Spülraum (110) angeordneten Sprüharm (120), einem Medienverteiler (130) mit Anschlüssen für die medizinischen Instrumente, und mit einer Leitung (160) zum Leiten des Pflegemittels von der Vorrichtung (170) zu dem Medienverteiler (130).
